# EUROPEAN PATENT SPECIFICATION

(11) **EP 0 919 621 B1**
(45) Date of publication and mention of the grant of the patent: **29.10.2008**
(21) Application number: 97908525.5
(22) Date of filing: 27.03.1997
(51) Int. Cl.: C12N 15/54, C12P 21/02, C12N 9/12, C12N 1/21

(54) **HUMAN TAK1 AND DNA ENCODING THE SAME**
MENSCHLICHE TAK1 UND FÜR DIESE KODIERENDE DNA
TAK1 HUMAINE ET ADN CODANT CELLE-CI

(30) Priority: 24.07.1996 US 685625; 27.09.1996 JP 25674796
(43) Date of publication of application: 02.06.1999
(73) Proprietor: CHUGAI SEIYAKU KABUSHIKI KAISHA, Tokyo 115 (JP)
(72) Inventor: MATSUMOTO, Kunihiro, Nagoya-shi, Aichi 464 (JP); IRIE, Kenji, Nagoya-shi, Aichi 466 (JP)
(74) Representative: HOFFMANN EITLE
(86) International application number: PCT/JP1997/001050
(87) International publication number: WO 1998/003663

(56) References cited:
- SAKURAI, H. ET AL.: "TGF-beta-Activated Kinase 1 Stimulates NF-kappaB Activation by an NF-kappaB-Inducing Kinase-Indepemdent Mechanism" BIOCHEMICAL AND BIOPHYSICAL RESEARCH COMMUNICATIONS, vol. 243, no. 2, 13 February 1998 (1998-02-13), pages 545-549, XP000867678
- SCIENCE, 270, 1995, K. YAMAGUCHI et al., "Identification of a Member of the MAPKKK Family as a Potential Mediator of TGF-beta Signal Transduction", p. 2008-2011.
- HYUGA SAITO et al., "New Molecular Genetics for Bio-Science (in Japanese)", (NANKODO), 1987, p. 235-236.
- CELL, 80, 1995, C.J. MARSHALL, "Specificity of Receptor Tyrosine Kinase Signaling: Transient Versus Sustained Extracellular Signal-Regulated Kinase Activation", p. 179-185.
- SCIENCE, 265, 1994, J.F. SMOTHERS et al., "Stimulatory Effects of Yeast and Mammalian 14-3-3 Proteins on the Raf Protein Kinase", p. 1716-1719.
- SCIENCE, 241, 1988, STEVEN K. HANKS et al., "The Protein Kinase Family: Conserved Features and Deduced Phylogeny of the Catalytic Domains", p. 42-52.
- NATURE, 324, 1986, RANDALL K. SAIKI et al., "Analysis of Enzymatically Amplified beta-Globin and HLA-DQalpha DNA with Allete-Specific Oligonucleotide Probes", p. 163-166.
- MAEDA, T. ET AL.: "Activation of Yeast PBS2 MAPKK by MAPKKKs or by Binding of an SH3-Containing Osmosensor" SCIENCE, vol. 269, 28 July 1995 (1995-07-28), pages 554-558,
- DATABASE EMBL [Online] 29 January 1996 (1996-01-29), MATSUMOTO, K.: "Mouse mRNA for TAK1 (TGF-beta.activated kinase), complete cds." Database accession no. D76446
- SHIBUYA, H. ET AL.: "An Activator of the TAK1 MAPKKK in TGF-beta Signal Transduction" SCIENCE, vol. 272, 24 May 1996 (1996-05-24), pages 1179-1182,
- KEETON, M.R. ET AL.: "Identification of Regulatory Sequences in the Type 1 Plasminogen Activator Inhibitor Gene Responsive to Transforming Growth factor beta" JOURNAL OF BIOLOGICAL CHEMISTRY, vol. 266, 5 December 1991 (1991-12-05), pages 23048-23052,

## Description

### Technical Field

Disclosed is a kinase (transforming growth factor-β (TGF-β) activated kinase 1; TAK1) carrying the signal transduction system of the TGF-β family, said kinase being activated by TGF-β, a method for producing the kinase, and a human gene encoding the kinase. TAK1, also referred to as the activator of MAPK kinase (AMK-1), is an enzyme that is activated by TGF-β and BMP (bone morphogenetic protein) and that phosphorylates and thereby activates the MAPK kinase.

### Background Art

Receptors of the TGF-β superfamily contain Ser/Thr kinase in the intracellular domain and are divided into type I that has a repeated sequence of Gly and Ser (GS box) at the amino-terminal end near the transmembrane domain and type II that does not have the GS box. It is believed, in the case of TGF-β, that it forms a complex with the type I receptor after a ligand has bound to the type II receptor, in which a constitutively phosphorylated kinase of the type II receptor phosphorylates the vicinity of GS box of the type I receptor, thereby activating the type I receptor so that a signal from the above ligand is transmitted into the cell. However, little is known about signaling molecules downstream of this receptor.

For a eukaryotic budding yeast Saccharomyces cereviceae, it is known, as a signal transduction cascade in which an extracellular mating pheromone causes conjugation, that the mating pheromone activates a G-protein, which in turn activates a MAPKK kinase (MAPKKK) (Ste11), and the activated MAPKKK phosphorylates and activates a MAPK kinase (MAPKK), and then the thus activated MAPKK (Ste7) phosphorylates and activates a MAP kinase (mitogen-activated protein kinase; MAPK), and finally the MAPK activates FUS1 protein to initiate cell conjugation.

As such a MAPKKK, TAK1 (TGF-β activated kinase 1) derived from mouse has been known so far (K. Yamaguchi et al., Science (1995) 270, 2008-2011).

### Disclosure of Invention

The present invention intends to provide a novel factor in the signaling system of the receptor of mammalian TGF-β said factor being located downstream of said receptor and being involved in said signaling system; gene encoding said factor; and a method for producing said factor.

In efforts to solve the above problems, the applicants of the present invention have succeeded in inserting a human-derived cDNA into a yeast Saccharomyces cereviceae that is deficient in MAPKKK activity (Ssk2/Ssk22, Shol) in the signal transduction cascade of the above mating pheromone, screening for cDNAs that can complement the activity-deficient MAPKKK, and cloning cDNAs that can complement the activity-deficient MAPKKK, and thereby have accomplished the present invention.

Thus, it is disclosed a polypeptide having a kinase activity that is activated by transforming growth factor (TGF)-β, said polypeptide comprising an amino acid sequence from Ser at position 23 to Ser at position 579 set forth in SEQ ID NO: 5.

It is disclosed a polypeptide having a kinase activity that is activated by TGF-β, said polypeptide comprising an amino acid sequence from Met at position 1 to Ser at position 579 set forth in SEQ ID NO: 5.

It is also disclosed DNA encoding a polypeptide having a kinase activity that is activated by TGF-β, said polypeptide comprising an amino acid sequence from Ser at position 23 to Ser at position 579 set forth in SEQ ID NO: 5.

It is also disclosed DNA encoding a polypeptide having a kinase activity that is activated by TGF-β, said DNA comprising a nucleic acid sequence from T at position 249 to A at position 1919 set forth in SEQ ID NO: 5.

Also disclosed is DNA encoding a polypeptide having a kinase activity that is activated by TGF-β, said polypeptide comprising an amino acid sequence from Met at position 1 to Ser at position 579 set forth in SEQ ID NO: 5.

Also disclosed is DNA encoding a polypeptide having a kinase activity that is activated by TGF-β, said DNA comprising a nucleic acid sequence from A at position 183 to A at position 1919 set forth in SEQ ID NO: 5.

The present invention also provides a vector comprising any of the above-mentioned DNAs, a host cell transformed with a vector comprising any of the above-mentioned DNAs, and a method for producing a polypeptide having a kinase activity that is activated by TGF-β, which method comprises culturing a host cell transformed with a vector comprising any of the above-mentioned DNAs and then recovering a product from the culture.

Also disclosed is a polypeptide having a kinase activity that is activated by TGF-β, said polypeptide being produced by the above method, and a kinase that is activated by TGF-β, said kinase comprising an amino acid sequence from Ser at position 23 to Ser at position 579 set forth in SEQ ID NO: 5.

The present invention also provides a fusion protein of the above polypeptide, protein and another protein. Brief Explanation of the Drawings

Fig. 1 shows a yeast expression vector pNV11.

Fig. 2 is a graph showing the effects of TGF-β addition on the expression of various TAK1 genes evaluated using a luciferase gene as a reporter gene.

Fig. 3 is a graph showing the effects of TGF-β and BMP-4 on the activity of a TAK1 gene in MC3T3-E1 cells as measured by an immunoprecipitation method and a coupled kinase method.

Fig. 4 is a graph showing the effects of various concentrations of TGF-β or BMP-4 on TAK1 kinase activity in the cells transfected with the HA-TAK1 gene. TAK1ΔN shows a result when the cells transfected with the TAK1ΔN gene were not stimulated by either TGF-β or BMP-4.

Fig. 5 shows a comparison of the base sequence of DNA encoding mouse TAK1 and that of DNA encoding human TAK1.

Fig. 6 shows a comparison of the base sequence of DNA encoding mouse TAK1 and that of DNA encoding human TAK1.

Fig. 7 shows a comparison of the base sequence of DNA encoding mouse TAK1 and that of DNA encoding human TAK1.

Fig. 8 shows a comparison of the base sequence of DNA encoding mouse TAK1 and that of DNA encoding human TAK1.

Fig. 9 shows a comparison of the base sequence of DNA encoding mouse TAK1 and that of DNA encoding human TAK1.

Fig. 10 shows a comparison of the amino acid sequence of mouse TAK1 and that of human TAK1.

Fig. 11 shows a comparison of the amino acid sequence of mouse TAK1 and that of human TAK1.

### Embodiment for Carrying out the Invention

In accordance with the present invention, the cloning of the desired gene can be detected by inserting, for example, an expression vector comprising a mammalian cDNA into a yeast that is deficient in the MAPKKK activity and that has a readily detectable reporter gene at the terminal of the cascade, ant then by expressing said reporter gene to detect the introduction of cDNA that complements the deficient MAPKKK activity. Moreover, another yeast can be used that is deficient in Ssk2/Ssk22 and Sho1 activity and that functions, for example, in a high osmotic pressure-signaling system.

As such a detection system, there can be used a MAPK route that transmits a signal of the mating pheromone in Saccharomyces cereviceae (I. Herskowitz, Cell, Vol. 80, 187 (1995); D.E. Lein et al., Curr. Opin. Cell Biol. Vol. 7, 197 (1995); J. Schulz et al., Curr. Opin. Gene Dev., Vol. 5, 31 (1995)). The normal signal transduction cascade in this system consists of Ste11 kinase, Ste7 kinase, and Fus3/Kss1 kinase, which correspond to MAPKKK, MAPKK, and MAPK, respectively. Ste11, Ste7, and Fus3/Kss1 sequentially act to thereby transmit signals to the transcription factor Ste12, which Ste12 in turn activates the transcription of a mating-specific gene such as FUS1.

With respect to the screening of cDNA, there can be used a cascade that has a functional mutation of Ste7 (STE7^{P368}) and a deficiency mutation of Ste11 (Ste11Δ) in the above cascade (K. Irie et al., Science Vol. 265, 1716 (1994)). In this system, it has been confirmed that a mammalian Raf or an activated type of MEKK (FafΔN or MEKKΔN, respectively) complements the deficiency of Ste11 activity in a Ste7^{P368}-dependent manner, when it is monitored by a histidine phenotype (His) imparted by the reporter gene FUS1p::HIS3 corresponding to the mating route. Thus, by introducing the subject cDNA into a yeast having the above mutated cascade and by detecting the histidine phenotype, cDNA that can complement Ste11Δ (MAKKK deficiency) can be selected.

As the subject cDNA, there can be used a cDNA library of any mammalian origin. For example, a cDNA expression library from a mouse cell line such as the mouse cell line BAF-B03. This cDNA library can be obtained by cloning cDNA corresponding to poly (A)-RNA from a mouse IL-3-dependent pro-β cell line BAF-B03 under the control of TDH3 promoter of a yeast expression vector pNV11. Another example of the subject cDNA library to be used is a human cell line such as a cDNA expression library from a human cell line Jurkat.

One positive clone was obtained by screening the above cDNA library using the above screening system. The base sequence of cDNA of this clone and the amino acid sequence encoded by the cDNA correspond to the nucleotide numbers 223 to 1893 and the amino acid numbers 23 to 579 of SEQ ID NO: 1.

cDNA libraries from a human cell line can be screened by the above screening system. Alternatively, cDNA libraries from a human cell line can be screened using a mouse cDNA as a probe in the manner described above.

The cDNA of another positive clone and the amino acid sequence encoded by the cDNA correspond to the nucleotides 249 to 1919 and the amino acids 23 to 579 set forth in SEQ ID NO: 5.

In order to obtain a longer cDNA (full-length cDNA), the above cDNA libraries were screened using said cDNA as a probe to thereby obtain multiple positive clones. These clones had a 5'-extension of approximately 230 bp to said cDNA. cDNA that contains this 5'-extension was designated as TAK1 cDNA and cDNA that was cloned first and that does not contain this 5'-extension was designated as TAK1ΔN cDNA. The nucleotide sequence of TAK1 cDNA is shown in 1 to 2443 of SEQ ID NO: 1, and the amino acid sequence encoded by the cDNA is shown in the amino acid numbers 1 to 579 of SEQ ID NO: 1. The protein or the polypeptide represented by the amino acid sequence is designated as TAK1 protein or polypeptide. In contrast, the protein or the polypeptide represented by the amino acid sequence encoded by TAK1 ΔN cDNA is designated as TAK1 ΔN protein or polypeptide. Furthermore, the nucleotide sequence of human TAK1 cDNA is represented by the nucleotides 1 to 2656 and the amino acid sequence encoded by it is represented by the amino acids 1 to 579 of SEQ ID NO: 5.

The primary sequence of the TAK1 protein suggests that this protein has a protein kinase catalysis domain at the N-terminal end and a C-terminal domain of about 300 amino acid residues. This catalysis domain contains a consensus sequence corresponding to the protein kinase subdomains I to XI (S.K. Hanks et al., Science 241, 42 (1988)). This catalysis domain has an about 30% identity with the amino acid in the Raf-1 (T.I. Bonner et al., Nucleic Acids Res. Vol. 14, 1009 (1986)) and MEKK (C.A. Langer-Carter et al., Science Vol. 260, 315 (1993)). The sequence of the 300 amino acid residues at the C-terminal following the above catalysis domain does not have a conspicuous homology with other proteins.

TAK1 ΔN cDNA deficient in the codons of 22 amino acids at the N-terminal which has been introduced into a yeast having a ste11Δ mutation can complement the ste11Δ mutation (MAPKKK deficiency), but the full-length TAK1 cDNA introduced into a ste11 Δ mutant does not complement the ste11Δ mutation. It is believed, therefore, that TAK1 kinase is activated by the removal of 22 the amino acids in the N-terminal.

Thus, the present invention provides DNA encoding a polypeptide comprising an amino acid sequence from Met at position 1 to Ser at position 579 set forth in SEQ ID NO: 5. This DNA includes, as a typical example, DNA encoding a polypeptide comprising an amino acid sequence from the amino acid Ser at position 23 to the amino acid Ser at position 579, and DNA encoding a polypeptide comprising an amino acid sequence from an amino acid Glu at position 30 to an amino acid Asp at position 295. However, the DNA may also include DNA encoding a polypeptide comprising an amino acid sequence from any of the amino acids from Met at position 1 to Glu at position 30 to the amino acid Asp at position 295.

Because it is believed that even from DNA encoding a polypeptide having an extended N-terminal, an active enzyme could be obtained by processing of the polypeptide after expression, and that it will have a similar kinase activity even without regions other than the kinase at the C-terminal.

Also disclosed are polypeptides or proteins, especially polypeptides or proteins retaining the TAK1 activity, having the amino acid sequences corresponding to the nucleotide sequences of various DNAs mentioned above. As a more specific example, the present invention relates to polypeptides or proteins expressed by introducing various DNAs mentioned above as they are inserted into, for example a vector, especially an expression vector, into host cells such as animal cells or microorganism cells.

Typically, the polypeptide or the protein of the present invention has an amino acid sequence from any of an amino acid from Met at position 1 (inclusive) to Ser at position 23 (inclusive) to the amino acid Ser at position 579 of SEQ ID NO: 5.

The present invention also provides a fusion protein of the above polypeptide or protein and another protein. Anther protein that is fused with a polypeptide or a protein having the TAK1 activity may be chosen as appropriate in addition to hemagglutinin mentioned in examples. DNA encoding a fusion protein of a polypeptide or a protein having the TAK1 activity with another protein can be constructed and expressed by the method set forth in Example 4.

As mentioned earlier, cDNA encoding human TAK1 can be obtained using cDNA encoding mouse TAK1; Examples 5 and 6 describe the isolation of cDNA encoding human TAK1.

The various DNAs mentioned above can be cloned from an animal cell as, for example, cDNA by the method set forth in Example 2. Mutated or modified DNA as compared to the original cDNA can be prepared using the original cDNA as a template in a conventional method such as PCR amplification and site-specific mutagenesis.

The polypeptides or proteins of the present invention can be obtained by expressing the corresponding DNA in a suitable host cell. As the host cell in this case, there may be used cultured cells of higher eukaryotic cells such as human, monkey, mouse, hamster, and frog cells, for example THP-1 cells, MC3T3-E1 cells, XTC cells, MvILu cells, CHO cells, and COS cells; cultured cells of lower eukaryotic cells including, for example, fungi such as the fungi of genus Aspergillus such as Aspergillus niger; or yeasts including, for example, the yeasts of genus Saccharomyces such as Saccharomyces cereviceae, and the like. As the host cell, furthermore, prokaryotic cells including, for example, bacteria such as Escherichia coli may be used.

When the desired DNA is to be expressed in these hosts, an expression-regulating sequence such as a suitable promoter is used depending on the host. In the expression in animal cells, for example, a plasmid containing each of promoters such as pCDM8, pSV, and pEP is used, and in yeast hosts, a plasmid such as pNV11 is used, and in Escherichia coli, a plasmid such as pGEMEX and pUEX is used.

Transformed hosts may be cultured in a conventional method. The polypeptides or proteins of the present invention can be produced using a transgenic animal (Glaser, V., SPECTRUM Biotechnology Applications, 1993) and an insect such as silkworm (Maeda, S. et al., Nature (1985) 315, 592-594) as a host. Recovery and/or purification of polypeptides and proteins thus produced may be accomplished by a commonly used method for enzyme purification such as centrifugation, filtration, gel filtration chromatography, and affinity chromatography.

Kinases activated by TGF-β that carries the signal transduction system of the TGF-β family are useful for use in the search of drugs that inhibit or promote signaling of TGF-β and its superfamily known to be involved in various diseases.

### Examples

The present invention will now be explained in further details with reference to the following examples.

### Example 1. Construction of cDNA library

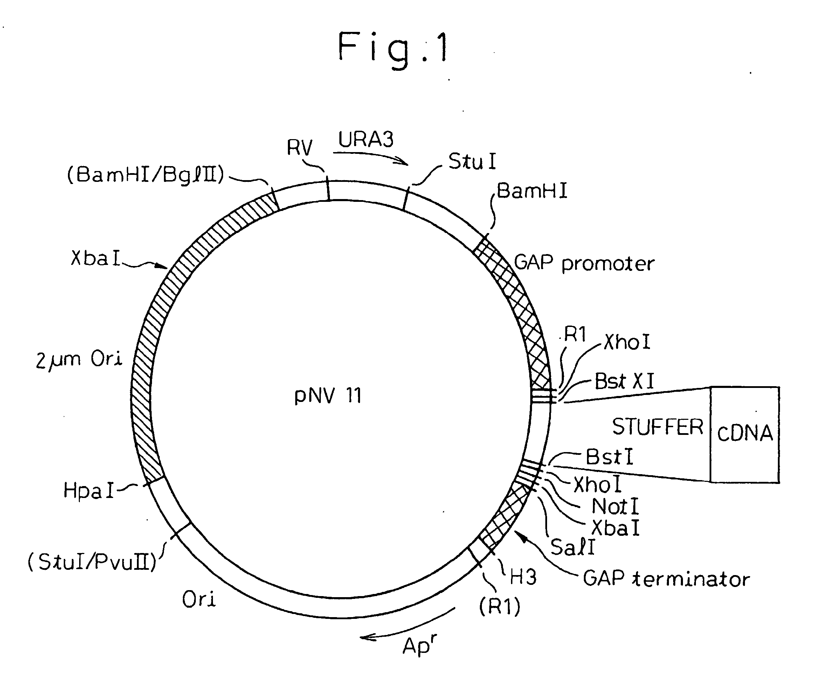

cDNA was synthesized from poly(A)-RNA from a mouse IL-3-dependent cell line BAf-BO3 according to a conventional method and was then inserted into a yeast expression vector pNV11 (Ninomiya-Tsuji, J. et al., Proc. Natl. Acad. Sci. U.S.A. 88, 9006-9010 (1991)) shown in Fig. 1 under the control of the TDH3 promoter to construct a cDNA library.

### Example 2. Screening of cDNA library

A cDNA library prepared in Example 1 was screened using Saccharomyces cereviceae SY1984-P (his3Δ, ste11Δ, FUS1p::HIS3, STE7P368). In this yeast, Ste11 has been mutated and the activity is deficient in the signal transduction system of mating pheromone, and the FUS1 upstream activation sequence has been ligated to the HIS3 open reading frame to form a reporter gene. The yeast strain is deficient in the original his3 and thus it can grow only when exogenous histidine is present in the culture medium or when the mutation-derived deficiency in the Ste11 activity has been complemented.

S. cerevisiae SY1984-P was transformed with various plasmids. The plasmids used were YCplac22 (vector), pRS314PGKMEKKCT (expresses MEKKΔN (K.J. Blumer et al., Proc. Natl. Acad. Sci. U.S.A. Vol. 91, 4925 (1994) that lacks the N-terminal domain downstream of the PGK1 promoter), and pADU-RafΔN (expresses RafΔN (K.Irie et al., Science Vol. 265 1716 (1994) that lacks the N-terminal domain downstream of the ADH1 promoter). These transformants were plated onto a SC-His plate that lacks histidine and were incubated at 30°C. As a result, the yeast that was transformed with the YCplac22 vector did not grow but the yeast transformed with pRS314PGKMEKKCT or pADU-RafΔN did. This confirmed that the screening system is effective.

Then the above screening system yeast strain YS1984-P was transformed with the cDNA library constructed in Example 1 and then was screened on an SC-His plate to obtain one positive clone pNV11-HU11. The cDNA of this clone was designated as TAK1ΔN cDNA. The nucleotide sequence of this cDNA was determined by the dideoxy nucleotide chain termination method. The nucleotide sequence corresponds to the sequence of nucleotides 223 to 1893 set forth in SEQ ID NO: 1 and the amino acid sequence encoded by it corresponds to Ser at position 23 to Ser at position 579 of the amino acid sequence set forth in SEQ ID NO: 1.

Then, in order to clone the full-length cDNA, the above TAK1ΔN cDNA was radiolabeled and used as a probe to further screen the cDNA library obtained in Example 1. Thus, multiple positive clones were obtained. The cDNA of this clone was subcloned into the EcoRI site of pBS vector (manufactured by Stratagene) to obtain pGS-TAK1-5'. This clone was a full-length clone containing the initiation codon ATG. The cDNA was designated as TAK1 cDNA. The nucleotide sequence thereof is shown set forth in SEQ ID NO: 1. The full-length amino acid sequence of Met at position 1 to Ser at position 579 has been encoded in nucleotides 1 to 2443 in the above sequence.

### Example 3. Tissue distribution of TAK1 gene

Total RNA was extracted from various tissues of mice and was subjected to Northern blotting using the above radiolabeled TAK1 cDNA as a probe to find that RNA that hybridizes with TAK1 cDNA was expressed in all the tissues or organs tested (spleen, thymus, lung, heart, liver and brain). It was present at high levels in the spleen, thymus, and brain, and at low levels in the lung, heart, and the liver.

### Example 4. Properties of TAK1 kinase

In order to study the functions of the kinase activated by TGF-β in mammalian cells, TAK1 cDNA and TAK1ΔN cDNA were inserted into a mammalian expression vector pEF (H. Shibuya et al., Nature Vol. 357, 700 (1992)) under the control of the human elongation factor (EF) promoter to obtain expression plasmids pEF-TAK1 and pEF-TAK1ΔN. The expression plasmids pEF-TAK1 and pEF-TAK1ΔN contain a full-length TAK1 coding sequence and TAK1ΔN coding sequence, respectively, under the control of the EF promoter.

Thus, 2.3 kb of XhoI fragment of pNV11-HU11 was inserted into a XhoI gap of pBS to obtain pBS-TAK1ΔN. pEF-MSS1 (H. Shibuya et al., Nature Vol. 357, 700 (1992)) was cleaved with EcoRI and XbaI, into which were introduced a synthetic EcoRI-XhoI linker (sense strand: 5'-AATTCGCCACCATGGC-3') (SEQ ID NO: 2); antisense strand: 5'-TCGAGCCATGGTGGCG-3') (SEQ ID NO: 3) (containing the initiation codon ATG), and an XhoI-HindIII fragment and a HindIII-XbaI fragment from pBS-TAK1ΔN to construct pEF-TAK1ΔN. pBS was cleaved with EcoRI and XhoI, into which were inserted an EcoRI-SacI fragment from pBS-TAK1Δ-5' and a SacI-XhoI fragment from pBS-TAK1ΔN to obtain pBS-TAK1 containing the full-length cDNA of TAK1 (TAK1 cDNA). pEF-MSS1 was cleaved with EcoRI and SalI, into which was inserted an EcoRI-SacI fragment from pBS-TAK1 to construct pEF-TAK1.

E. coli having the plasmid pEF-TAK1 has been internationally deposited under the provisions of the Budapest Treaty as Escherichia coli MC1061/P3 (pEF-TAK1) and E. coli having the plasmid pEF-TAK1ΔN as Escherichia coli MC1061/P3 (pEF-TAK1ΔN) on September 29, 1995 with the National Institute of Bioscience and Human Technology, the Agency of Industrial Science and Technology, of 1-3, Higashi 1-chome, Tsukuba city, Ibalaki pref., Japan, as FERM BP-5246 and FERM BP-5245, respectively.

The TAK1 gene contained in the plasmid pEF-TAK1 can be excised using an appropriate restriction enzyme such as EcoRI and BamHI.

A study of the effects of TAK1 on the induction of gene expression by various ligands has revealed that TAK1 has an effect on gene induction by TGF-β. An initial cellular response to TGF-β induces an elevation in the mRNA level of plasminogen activator inhibitor 1 (PAI-1) (M.R. Keeton et al., J. Biol. Chem. Vol. 266, 23048 (1991)).

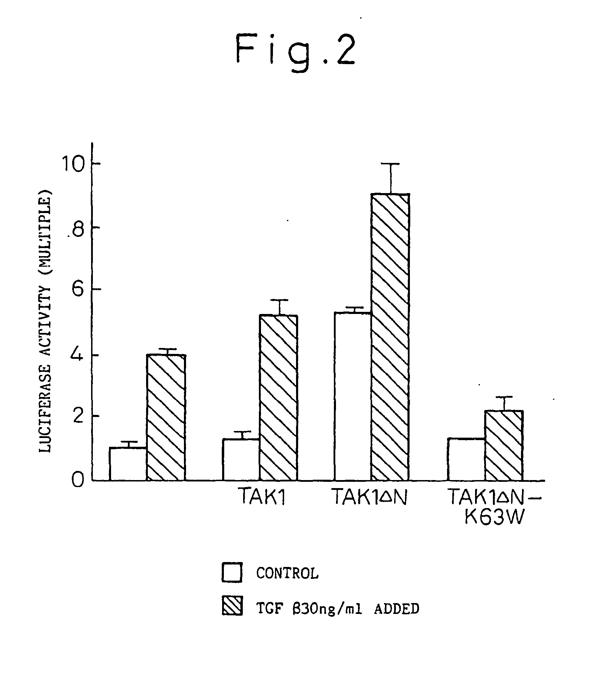

In order to study the effects of TAK1 on TGF-β response, TGF-β reporter plasmid p800neoLUC containing a luciferase gene regulated by the PAI-1 promoter induced by TGF-β (M. Abe et al., Analyt. Biochem., Vol. 216, 276 (1994)) was transiently transfected to MvlLu lung epithelial cells by the calcium phosphate method (H. Shibuya et al., Nature Vol. 357, 700 (1992)). In this method of measurement, the luciferase activity induced by TGF-β can be measured by the transfection of p800neoLUC onto the MvlLu lung epithelial cells. The MvlLu cells transiently transfected by p800neoLUC responded to TGF-β with a 4- to 5-fold enhanced reporter gene activity. This result is shown in the vector section of Fig. 2.

The previously constructed TAK1 or TAK1ΔN expression plasmid was transiently transfected into MvlLu cells. Expression of TAK1 slightly enhanced the expression of TGF-β -derived genes and TAK1ΔN constitutively activated the expression of the PAI-1 gene (the section of TAK1ΔN of Fig. 2). The level of constitutive expression of a reporter gene by TAK1ΔN is equal to that in a transfectant treated with TGF-β. Thus, the activated TAK1 (i.e. TAK1ΔN) can transfer signals in the absence of TGF-β. Furthermore, when TGF-β was added to a TAK1ΔN transfectant, the expression of the PAI-1 gene was further enhanced.

In Fig. 2, the open bars represent a case in which no induction of TGF-β was conducted, and the shaded bars represent a case in which the induction of TGF-β was conducted. In the above experiment, cells after transfection were cultured for 20 hours in the presence or absence of human TGF-β1 (30 ng/ml) to prepare an extract from the cells, and luciferase was measured as described in H. Shibuya et al., Mol. Cell. Biol. Vol. 14, 5812 (1994). In the graph of Fig. 2, relative luciferase activities of cells transformed by a vector (containing no TAK1 gene) are shown with the luciferase activity in the absence of TGF-β1 induction being set as 1. The results of bar graphs represent the mean of results of triplicate runs per experiment.

In order to confirm that the above results are mediated by the kinase activity of TAK1, a catalytically inactive TAK1ΔN-K63W was constructed. This was carried out by site-directed mutagenesis using PCR. In this vector, lysine at position 63 in the ATP binding site has been replaced with tryptophan. The mutation is expected to inactivate the kinase activity and signaling activity of TAK1ΔN. When TAK1ΔN-K63W is co-transfected with p800neoLUC, the ability of constitutively stimulating the expression of the PAI-1 gene was lost (Fig. 2). These results suggest that the kinase activity of TAK1ΔN is required for the TGF-β-independent expression of PAI-1 gene. Furthermore, a kinase-negative TAK1ΔN caused the partial reduction of TGF-β-induced expression. These results suggest that TAK1 may act as a mediator of a TGF-β-mediated route of signal transduction.

In order to obtain direct evidence that TAK1 functions in the TGF-β-mediated route of signal transduction, it was determined whether the treatment of cells with TGF-β could activate the kinase activity of TAK1. For the identification of an appropriate foreign substrate, an in vitro kinase reaction was conducted for TAK1 that was immunoprecipitated from yeast cells expressing TAK1 labeled with a hemagglutinin (HA) epitope (TAK1-HA) (a DNA sequence encoding an epitope recognized by anti-HA monoclonal antibody 12CA5 was ligated by PCR reaction to the 3'-terminal frame of DNA encoding TAK1).

The results of the immune complex kinase determination indicate that the activated form of TAK1 can phosphorylate and activate the XMEK2/SEK1 subfamily of MAPKK (B.M. Yasher et al., Nature Vol. 372, 794 (1994)). On the other hand, the phosphorylation of the original MAPKK-MEK1 (E. Nishida et al., Trends Biochem. Sci., 128 (1993); K.J. Blumer et al., ibid Vol. 19, 286 (1994); R.J. Davis, ibid Vol. 19, 470 (1990); C.L. Marchall, Cell, Vol. 80, 179 (1995)), histone and myelin basic protein was not detected. Thus, TAK1 kinase activity can be measured for its ability of activating XMEK2 in vitro.

Constructs for the expression of HA epitope-labeled TAK1 (HA-TAK1) were made in the following manner. A synthetic oligonucleotide encoding an HA epitope Tyr-Pro-Tyr-Asp-Val-Pro-Asp-Tyr-Ala (SEQ ID NO: 4) that is recognized by a monoclonal antibody 12CA5 was cloned into a SalI site (+3 position from the ATG codon) and an EcoRI site of pBS-TAK1 to construct pBS-HA-TAK1. pEF-MSS1 was cleaved with EcoRI and SalI, to which was inserted an EcoRI-XhoI fragment from pBS-HA-TAK1 to construct pEF-HA-TAK1.

In order to construct pBS-HA-TAK1ΔN, pNV11-HU11 was digested with XhoI and HindIII. The fragment obtained was isolated and inserted into a HincII-HindIII site of pBS-HA-TAK1. pEF-MSS1 was cleaved with EcoRI and SalI, into which was inserted a PstI-XhoI fragment from pBS-HA-TAK1ΔH to construct pBS-HA-TAK1ΔN. Both of these constructs have two copies of the N-terminal HA epitope expressed from the EF promoter.

These constructs pEF-HA-TAK1 or pEF-HA-TAK1ΔH were transiently transfected to the MC3T3-E1 mouse osteoblast (S. Ohta et al., FEBS Lett. Vol. 314, 356 (1992)). After stimulation by TGF-β1, the expressed HA-TAK1 was isolated by immunoprecipitation, and its activity was measured by the coupled kinase assay (S. Matsuda et al., J. Biol. Chem. Vol. 270, 12969 (1995)).

Thus, the transfected cells were treated with TGF-β1 (20 ng/ml) or BMP-4 (100 ng/ml) for 0 minute (untreated) to 30 minutes. The cells were scraped into a buffer solution (S. Matsuda et al., J. Biol. Chem. Vol. 270, 12781 (1995); T. Moriguchi et al., J. Biol. Chem. Vol. 270, 12969 (1995)), and the cellular extract was centrifuged at 15,000 x g for 10 minutes. The supernatant thus obtained was subjected to immunoprecipitation by anti-HA antibody. Thus, 300 µl aliquots of the above supernatant were mixed with 20 µl of antibody or 20 µl of protein A Sepharose, and the immune complex was washed twice with PBS, which was then used for kinase measurement (S. Matsuda et al., J. Biol. Chem. Vol. 270, 12781 (1995); T. Moriguchi et al., J. Biol. Chem. Vol. 270, 12969 (1995)).

Activity was expressed as an incremental multiple relative to the HA-TAK1 activity of non-stimulated cells. The activity of immunoprecipitated TAK1 was measured by its ability of activating recombinant XMEX2/SEK1 (S. Matsuda et al., J. Biol. Chem. Vol. 270, 12781 (1995); T. Moriguchi et al., J. Biol. Chem. Vol. 270, 12781 (1995)).

It has already been confirmed that HA-TAK1 does not directly phosphorylate KN-p38/MPK2. In accordance with the immunoblotting of each immunoprecipitate by anti-HA antibody, almost identical amounts of HA-TAK1 were recovered at each point of immunoprecipitation.

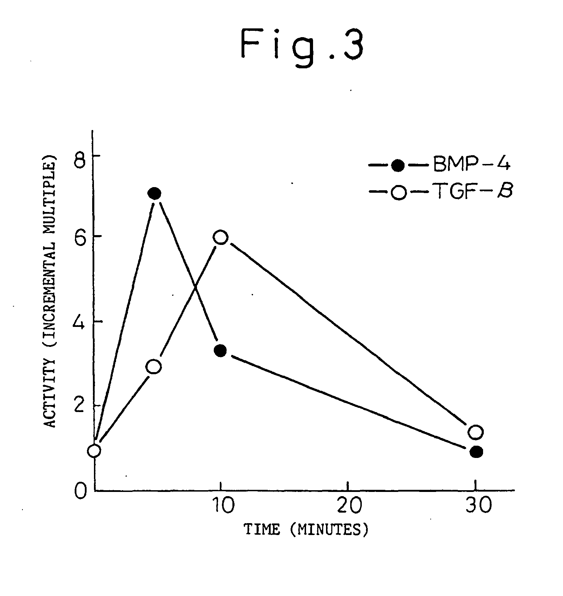

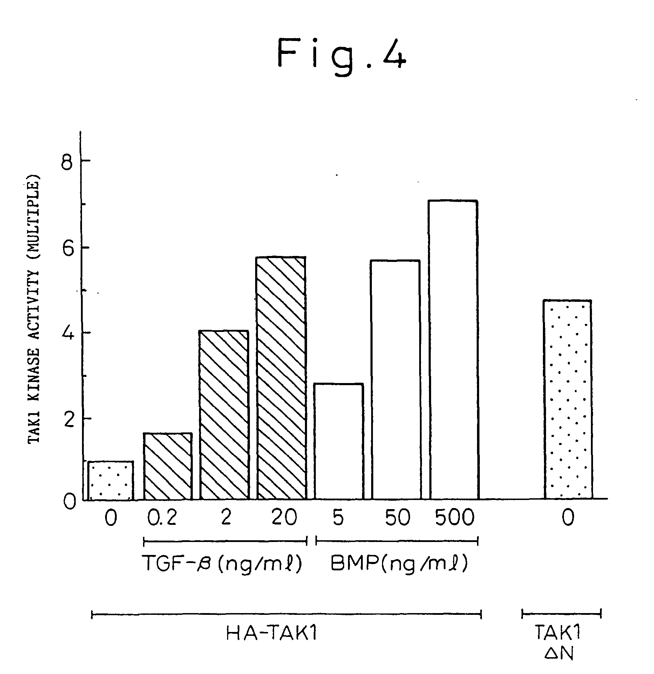

The result of the above experiment indicated that TAK1 kinase activity started to rise within five minutes after stimulation by TGF-β, reached a peak at 10 minutes, and returned to an almost baseline level within 30 minutes (Fig. 3). Moreover, TGF-β1 stimulated TAK1 kinase activity in a dose-dependent manner (Fig. 4). Then, it was determined whether TAK1 could be activated by BMP, a member of the TGF-β superfamily (A.H. Reddi et al., Curr. Opin. Genet. Dev. Vol. 4, 737 (1994)), or epithelial growht factor (EGF). Interestingly, BMP-4 also activated TAK1 kinase in a time- and dose-dependent manner (Fig. 4).

On the other hand, the activation of TAK1 was not observed in the cells treated with EGF. It is believed that the lack of TAK1 induction by EGF is not because MC3T3-E1 cells do not respond to EGF but because EGF signals are not mediated by TAK1. This is also apparent from the fact that EGF induces the expression of fos in MC3T3-E1 cells. Taken together, these data indicate that TAK1 is activated by the TGF-β superfamily.

TAK1ΔN can activate the expression of PAI-1 gene independently of TGF-β (Fig. 2), which suggests that TAK1ΔN protein has an enhanced kinase activity even in the absence of TGF-β treatment in the cell. In order to study this possibility, TAK1ΔN labeled with the HA epitope (HA-TAK1ΔN) (see above) was transiently transfected to MC3T3-E1 cells, and the activity of TAK1ΔN was determined by the immunocomplex kinase measurement. Thus, MC3T3-E1 cells were transfected by pEF-HA-TAK1ΔN, and from the transfected cells HA-TAK1ΔN was immunoprecipitated as described above and its activity was measured.

All the data are expressed as an incremental multiple from the HA-TAK1 activity of non-stimulated cells.

As shown in Fig. 4, TAK1ΔN protein has a considerably higher inherent kinase activity supporting the hypothesis that the TAK1ΔN lacking the 22 amino acid residues at the N-terminal are constitutively active.

### Example 5. Construction of cDNA library

From human T-cell cell line Jurkat cells, poly(A)RNA was prepared and cDNA was synthesized in a conventional method. This was inserted into a yeast expression vector pNV7 (Ninomiya-Tsuji, J. et al., Proc. Natl. Acad. Sci. U.S.A. 88, 9006-9010 (1991)) downstream of TDH3 promoter to construct a cDNA library.

### Example 6. Screening of cDNA library

A mutant Saccharomyces cereviceae lacking the activity of Ssk2/Ssk22 and Sho1 that act in the signal transduction system of high osmotic pressure stress can grow in the YEPD medium (Yeast extract 10 g/l, tryptone 20 g/l, glucose 20 g/l), but not in a medium with 1M sorbitol added thereto (T. Maeda et al., Science, 269, 554 (1995)). Therefore, by introducing cDNA into this mutant followed by screening, a cDNA that can complement the deficient Ssk2/Ssk22 activity can be isolated.

In fact, a Saccharomyces cereviceae strain deficient in the Ssk2/Ssk22 and Sho1 activity described in the above reference (ssk2Δ, ssk22Δ, sho1Δ) was transformed with pNV11-HU11 (mouse TAK1ΔN) obtained in Example 2. The transformant was plated to the YEPD plate containing 1M sorbitol and was incubated at 30°C for 30 minutes. As a result, the yeast transformed with pNV11-HU11 grew even under a high osmotic pressure stress. This confirmed that the screening system is effective.

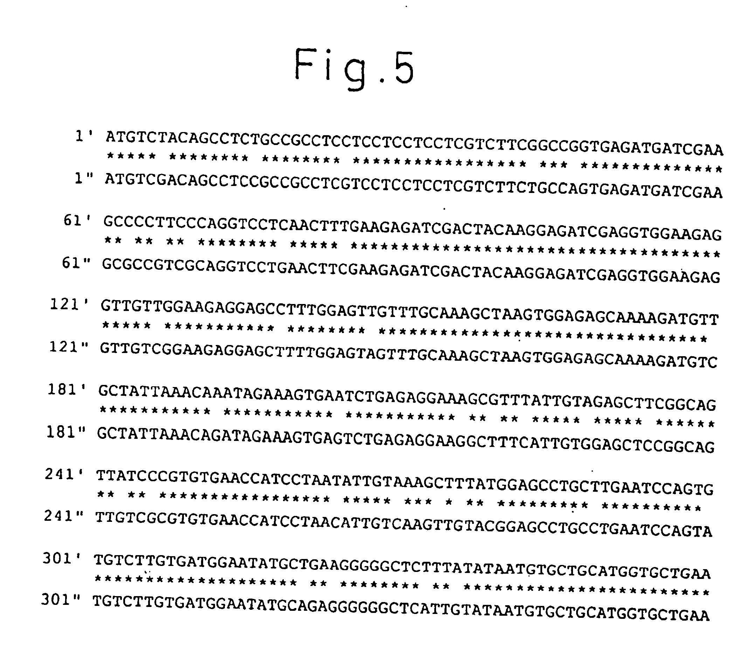

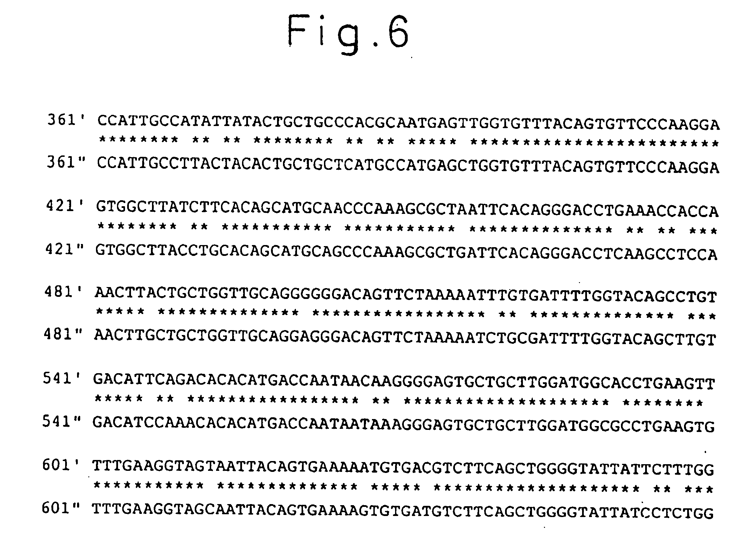

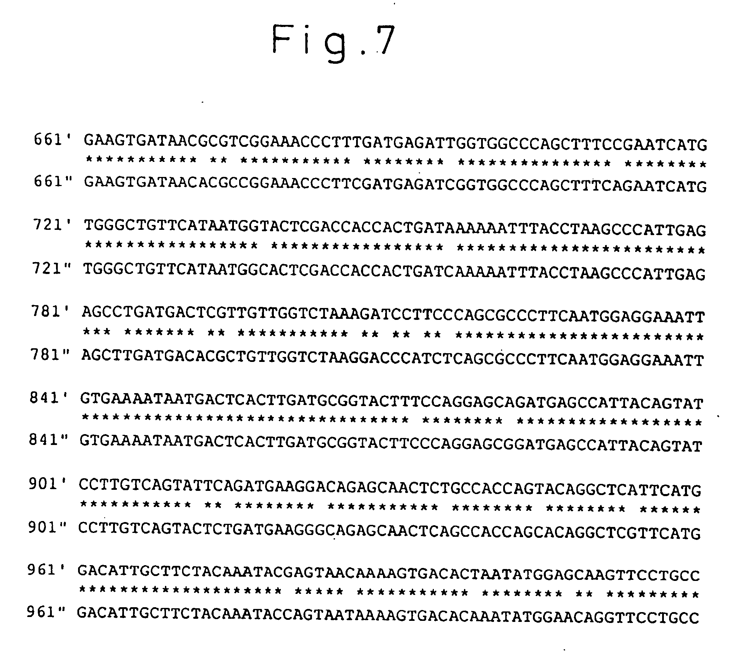

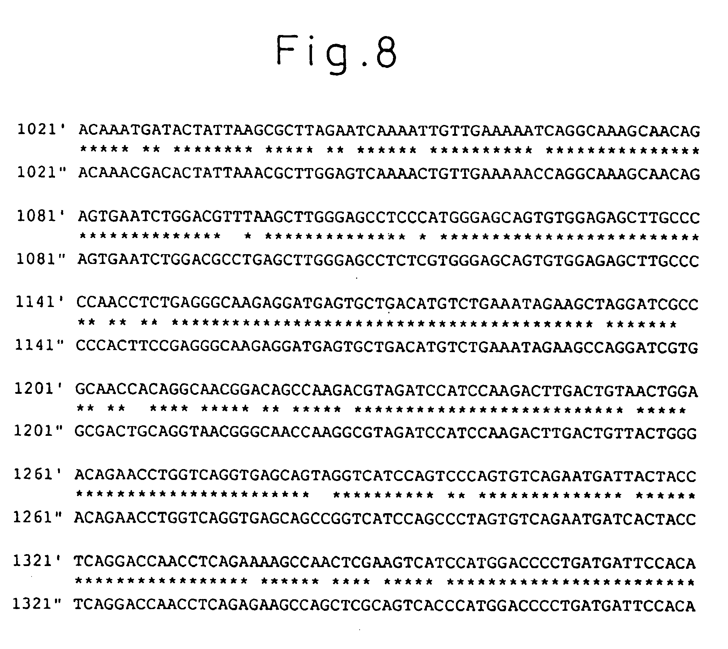

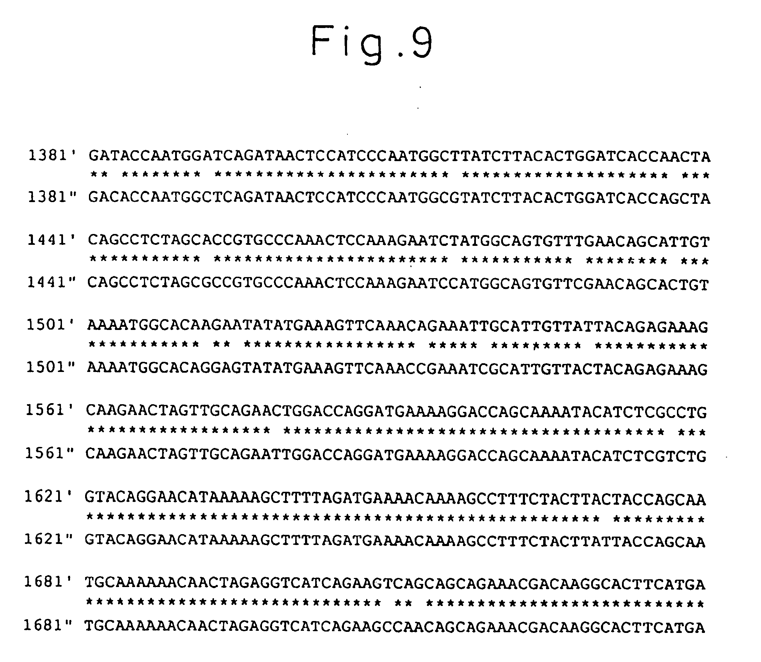

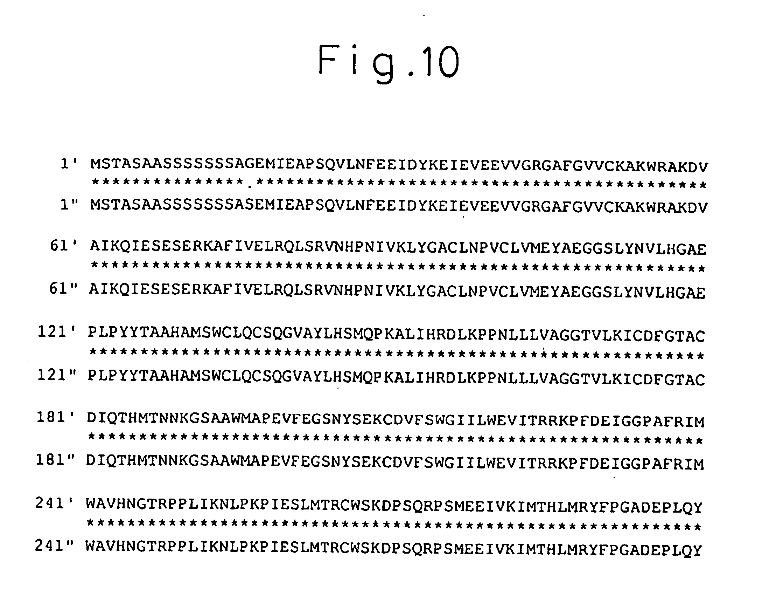

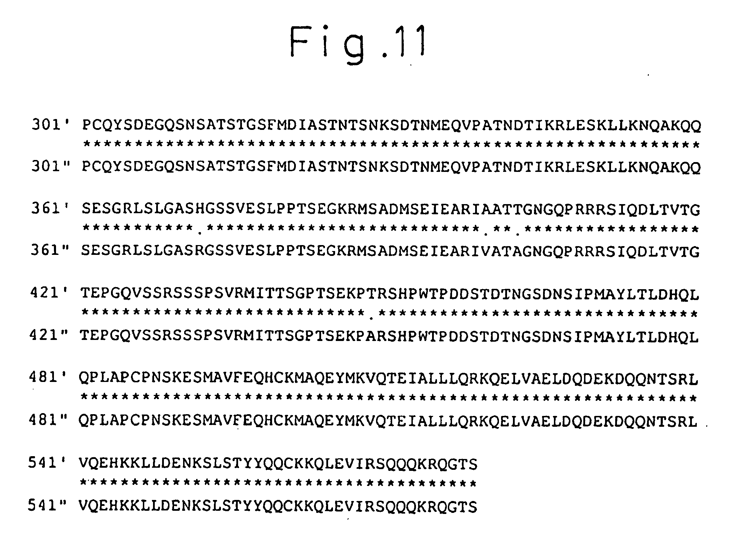

Then, this Saccharomyces cereviceae strain (ssk2Δ, ssk22Δ, sho1Δ) was transformed with the cDNA library constructed in Example 5, and was screened under a high osmotic pressure stress (incubated at 30°C in a YEPD medium containing 1M sorbitol). As a result, one positive clone pNV7-hTAK1 was obtained. cDNA contained in this clone was amplified using the PRISM Dye Terminator Cycle Sequencing kit (manufactured by Perkin Elmer) and the base sequence thereof was determined. The base sequence and the corresponding amino acid sequence were as set forth in SEQ ID NO: 5. The base sequence of this cDNA had a 92% homology with that of mouse TAK1, and the amino acid sequence encoded by the cDNA had a 99% homology with that of mouse TAK1. Comparisons of the base sequence of mouse TAK1 and human TAK1 are shown in Fig. 5 through Fig. 9, and those of the amino acid sequence are shown in Fig. 10 and Fig. 11.

Human TAK1 cDNA was subcloned into pUC19 that had been digested with SalI to obtain a plasmid phTAK1 containing the full-length cDNA of human TAK1. E. coli having the plasmid phTAK1 was internationally designated as Escherichia coli JM109 (phTAK1) under the provisions of the Budapest Treaty on July 19, 1996 with the National Institute of Bioscience and Human Technology, the Agency of Industrial Science and Technology, of 1-3, Higashi 1-chome, Tsukuba city, Ibalaki pref., Japan, as FERM BP-5598.

### Deposit of microorganisms

The following microorganisms were deposited with the Patent Microorganism Depository in the National Institute of Bioscience and Human Technology, the Agency of Industrial Science and Technology, of 1-3, Higashi 1-chome, Tsukuba city, Ibalaki pref., Japan, and were assigned the following accession numbers:
Organism: Escherichia coli MC1061/P3 (pEF-TAK1)
Date deposited: September 28, 1995
Accession number: FERM BP-5246
Organism: Escherichia coli MC1061/P3 (pEF-TAK1ΔN)
Date deposited: Septerber 28, 1995
Accession number: FERM BP-5245
Organism: Escherichia coli JM109 (phTAK1)
Date deposited: July 19, 1996
Accession number: FERM BP-5598

### SEQUENCE LISTINGS

SEQ ID NO: 1
   Sequence Length: 2443
   Sequence Type: Nucleic acid
   Strandedness: Single
   Topology: Linear
   Molecular Type: cDNA
   Sequence 
GAATTCGGCA CGAGGAGGAG CCGAAGCCGG GACTCGGCGG TGGCCCGGGT CGGTCCCGCG 60

CCACGGAGCG CCGGGCGGCG GGCTGCGGGG CTCCGGGCTG AAGGGCGCTG CGCGAGCCGG 120

AGGGCGGGCG CGGCCCCCCG GGCGCCGCGG GGGATC ATG TCG ACA GCC TCC GCC 174

Met Ser Thr Ala Ser Ala

1               5

GCC TCG TCC TCC TCC TCG TCT TCT GCC AGT GAG ATG ATC GAA GCG CCG 222

Ala Ser Ser Ser Ser Ser Ser Ser Ala Ser Glu Met Ile Glu Ala Pro

            10                  15                  20

TCG CAG GTC CTG AAC TTC GAA GAG ATC GAC TAC AAG GAG ATC GAG GTG 270

Ser Gln Val Leu Asn Phe Glu Glu Ile Asp Tyr Lys Glu Ile Glu Val

        25                  30                  35

GAA GAG GTT GTC GGA AGA GGA GCT TTT GGA GTA GTT TGC AAA GCT AAG 318

Glu Glu Val Val Gly Arg Gly Ala Phe Gly Val Val Cys Lys Ala Lys

    40                  45                  50

TGG AGA GCA AAA GAT GTC GCT ATT AAA CAG ATA GAA AGT GAG TCT GAG 366

Trp Arg Ala Lys Asp Val Ala Ile Lys Gln Ile Glu Ser Glu Ser Glu

55                  60                  65                  70
 
AGG AAG GCT TTC ATT GTG GAG CTC CGG CAG TTG TCG CGT GTG AAC CAT 414
Arg Lys Ala Phe Ile Val Glu Leu Arg Gln Leu Ser Arg Val Asn His
                75                  80                  85
CCT AAC ATT GTC AAG TTG TAC GGA GCC TGC CTG AAT CCA GTA TGT CTT 462
Pro Asn Ile Val Lys Leu Tyr Gly Ala Cys Leu Asn Pro Val Cys Leu
            90                  95                  100
GTG ATG GAA TAT GCA GAG GGG GGC TCA TTG TAT AAT GTG CTG CAT GGT 510
Val Met Glu Tyr Ala Glu Gly Gly Ser Leu Tyr Asn Val Leu His Gly
        105                 110                 115
GCT GAA CCA TTG CCT TAC TAC ACT GCT GCT CAT GCC ATG AGC TGG TGT 558
Ala Glu Pro Leu Pro Tyr Tyr Thr Ala Ala His Ala Met Ser Trp Cys
    120                 125                 130
TTA CAG TGT TCC CAA GGA GTG GCT TAC CTG CAC AGC ATG CAG CCC AAA 606
Leu Gln Cys Ser Gln Gly Val Ala Tyr Leu His Ser Met Gln Pro Lys
135                 140                 145                 150
GCG CTG ATT CAC AGG GAC CTC AAG CCT CCA AAC TTG CTG CTG GTT GCA 654
Ala Leu Ile His Arg Asp Leu Lys Pro Pro Asn Leu Leu Leu Val Ala
                155                 160                 165
GGA GGG ACA GTT CTA AAA ATC TGC GAT TTT GGT ACA GCT TGT GAC ATC 702
Gly Gly Thr Val Leu Lys Ile Cys Asp Phe Gly Thr Ala Cys Asp Ile
            170                 175                 180
CAA ACA CAC ATG ACC AAT AAT AAA GGG AGT GCT GCT TGG ATG GCG CCT 750
Gln Thr His Met Thr Asn Asn Lys Gly Ser Ala Ala Trp Met Ala Pro
        185                 190                 195
GAA GTG TTT GAA GGT AGC AAT TAC AGT GAA AAG TGT GAT GTC TTC AGC 798
Glu Val Phe Glu Gly Ser Asn Tyr Ser Glu Lys Cys Asp Val Phe Ser
    200                 205                 210
 
TGG GGT ATT ATC CTC TGG GAA GTG ATA ACA CGC CGG AAA CCC TTC GAT 846
Trp Gly Ile Ile Leu Trp Glu Val Ile Thr Arg Arg Lys Pro Phe Asp
215                 220                 225                 230
GAG ATC GGT GGC CCA GCT TTC AGA ATC ATG TGG GCT GTT CAT AAT GGC 894
Glu Ile Gly Gly Pro Ala Phe Arg Ile Met Trp Ala Val His Asn Gly
                235                 240                 245
ACT CGA CCA CCA CTG ATC AAA AAT TTA CCT AAG CCC ATT GAG AGC TTG 942
Thr Arg Pro Pro Leu Ile Lys Asn Leu Pro Lys Pro Ile Glu Ser Leu
            250                 255                 260
ATG ACA CGC TGT TGG TCT AAG GAC CCA TCT CAG CGC CCT TCA ATG GAG 990
Met Thr Arg Cys Trp Ser Lys Asp Pro Ser Gln Arg Pro Ser Met Glu
        265                 270                 275
GAA ATT GTG AAA ATA ATG ACT CAC TTG ATG CGG TAC TTC CCA GGA GCG 1038
Glu Ile Val Lys Ile Met Thr His Leu Met Arg Tyr Phe Pro Gly Ala
    280                 285                 290
GAT GAG CCA TTA CAG TAT CCT TGT CAG TAC TCT GAT GAA GGG CAG AGC 1086
Asp Glu Pro Leu Gln Tyr Pro Cys Gln Tyr Ser Asp Glu Gly Gln Ser
295                 300                 305                 310
AAC TCA GCC ACC AGC ACA GGC TCG TTC ATG GAC ATT GCT TCT ACA AAT 1134
Asn Ser Ala Thr Ser Thr Gly Ser Phe Met Asp Ile Ala Ser Thr Asn
                315                 320                 325
ACC AGT AAT AAA AGT GAC ACA AAT ATG GAA CAG GTT CCT GCC ACA AAC 1182
Thr Ser Asn Lys Ser Asp Thr Asn Met Glu Gln Val Pro Ala Thr Asn
            330                 335                 340
GAC ACT ATT AAA CGC TTG GAG TCA AAA CTG TTG AAA AAC CAG GCA AAG 1230
Asp Thr Ile Lys Arg Leu Glu Ser Lys Leu Leu Lys Asn Gln Ala Lys
        345                 350                 355
 
CAA CAG AGT GAA TCT GGA CGC CTG AGC TTG GGA GCC TCT CGT GGG AGC 1278
Gln Gln Ser Glu Ser Gly Arg Leu Ser Leu Gly Ala Ser Arg Gly Ser
    360                 365                 370
AGT GTG GAG AGC TTG CCC CCC ACT TCC GAG GGC AAG AGG ATG AGT GCT 1326
Ser Val Glu Ser Leu Pro Pro Thr Ser Glu Gly Lys Arg Met Ser Ala
375                 380                 385                 390
GAC ATG TCT GAA ATA GAA GCC AGG ATC GTG GCG ACT GCA GGT AAC GGG 1374
Asp Met Ser Glu Ile Glu Ala Arg Ile Val Ala Thr Ala Gly Asn Gly
                395                 400                 405
CAA CCA AGG CGT AGA TCC ATC CAA GAC TTG ACT GTT ACT GGG ACA GAA 1422
Gln Pro Arg Arg Arg Ser Ile Gln Asp Leu Thr Val Thr Gly Thr Glu
            410                 415                 420
CCT GGT CAG GTG AGC AGC CGG TCA TCC AGC CCT AGT GTC AGA ATG ATC 1470
Pro Gly Gln Val Ser Ser Arg Ser Ser Ser Pro Ser Val Arg Met Ile
        425                 430                 435
ACT ACC TCA GGA CCA ACC TCA GAG AAG CCA GCT CGC AGT CAC CCA TGG 1518
Thr Thr Ser Gly Pro Thr Ser Glu Lys Pro Ala Arg Ser His Pro Trp
    440                 445                 450
ACC CCT GAT GAT TCC ACA GAC ACC AAT GGC TCA GAT AAC TCC ATC CCA 1566
Thr Pro Asp Asp Ser Thr Asp Thr Asn Gly Ser Asp Asn Ser Ile Pro
455                 460                 465                 470
ATG GCG TAT CTT ACA CTG GAT CAC CAG CTA CAG CCT CTA GCG CCG TGC 1614
Met Ala Tyr Leu Thr Leu Asp His Gln Leu Gln Pro Leu Ala Pro Cys
                475                 480                 485
CCA AAC TCC AAA GAA TCC ATG GCA GTG TTC GAA CAG CAC TGT AAA ATG 1662
Pro Asn Ser Lys Glu Ser Met Ala Val Phe Glu Gln His Cys Lys Met
            490                 495                 500
 
GCA CAG GAG TAT ATG AAA GTT CAA ACC GAA ATC GCA TTG TTA CTA CAG 1710
Ala Gln Glu Tyr Met Lys Val Gln Thr Glu Ile Ala Leu Leu Leu Gln
        505                 510                 515
AGA AAG CAA GAA CTA GTT GCA GAA TTG GAC CAG GAT GAA AAG GAC CAG 1758
Arg Lys Gln Glu Leu Val Ala Glu Leu Asp Gln Asp Glu Lys Asp Gln
    520                 525                 530
CAA AAT ACA TCT CGT CTG GTA CAG GAA CAT AAA AAG CTT TTA GAT GAA 1806
Gln Asn Thr Ser Arg Leu Val Gln Glu His Lys Lys Leu Leu Asp Glu
535                 540                 545                 550
AAC AAA AGC CTT TCT ACT TAT TAC CAG CAA TGC AAA AAA CAA CTA GAG 1854
Asn Lys Ser Leu Ser Thr Tyr Tyr Gln Gln Cys Lys Lys Gln Leu Glu
                555                 560                 565
GTC ATC AGA AGC CAA CAG CAG AAA CGA CAA GGC ACT TCA TGATTCTCTG 1903
Val Ile Arg Ser Gln Gln Gln Lys Arg Gln Gly Thr Ser
            570                 575
GGACCGTTAC GTTTTAAAAT ATGCAAAGAC CTTTTTTTAA GAGAAGACAA ACCATTATAA 1963
CAGTTCATGA GTGTTAGCTT TTTGGCGTGT TCTGAATGCC AAATGCCTCT CTTTGCTGCA 2023
TTTGTTATGT CAGTTACCTT TCTTCTTATG GTGGATATAA AATCCACTGT CGTGTTGCAG 2083
CAGATGATGG CACCTGTGGC TTGGGAAGGC GAGSGTGCTC AGCTTCAGGG GCACATGAAG 2143
TGAACCTGGC TGTATGTGCA TGCTCCTGGA GTGAGCTACC TAACAGGAGG GGGTAGCACA 2203
CTGGCTACTG TGTGCAGGCA TCATCCTTTC TCTGTAGTAA AAGGTGGGAC CTCAAGAATT 2263
TTCTTCAAAG TGCTCATCTC AAAAATCTGA TTTTTTTCCC AGTAGATGGT ATGCTCCAAT 2323
GTAAAGACAG AGTATTAAAA TAACTTGTGG TACATTACAG AGGGACAGAA TGTTGAGGCT 2384
GAGTTCAAAG ACAGGGTTTG TGCCAACACA TCCTGGCTTT AGAGCACAAT GGATCTCGAG 2443
SEQ ID NO: 2
   Sequence Length: 16
   Sequence Type: Nucleic acid
   Strandedness: Single
   Topology: Linear
   Molecular Type: Synthetic DNA
   Sequence
      AATTCGCCAC CATGGC 16
SEQ ID NO: 3
   Sequence Length: 16
   Sequence Type: Nucleic acid
   Strandedness: Single
   Topology: Linear
   Molecular Type: Synthetic DNA
   Sequence
      TCGAGCCATG GTGGCG 16
SEQ ID NO: 4
   Sequence Length: 27
   Sequence Type: Amino acid
   Topology: Linear
   Molecular Type: Peptide
   Sequence 
Tyr Pro Tyr Asp Val Pro Asp Tyr Ala
1               5
SEQ ID NO: 5
   Sequence Length: 2656
   Sequence Type: Nucleic acid
   Strandedness: Single
   Topology: Linear
   Molecular Type: cDNA
   Sequence

GTCGAGATCC ATTGTGCTCT AAAGACGGCT GTGGCCGCTG CCTCTACCCC CGCCACGGAT 60

CGCCGGGTAG TAGGACTGCG CGGCTCCAGG CTGAGGGTCG GTCCGGAGGC GGGTGGGCGC 120

GGGTCTCACC CGGATTGTCC GGGTGGCACC GTTCCCGGCC CCACCGGGCG CCGCGAGGGA 180

TC ATG TCT ACA GCC TCT GCC GCC TCC TCC TCC TCC TCG TCT TCG GCC 227

Met Ser Thr Ala Ser Ala Ala Ser Ser Ser Ser Ser Ser Ser Ala

1               5                   10                  15

GGT GAG ATG ATC GAA GCC CCT TCC CAG GTC CTC AAC TTT GAA GAG ATC 275

Gly Glu Met Ile Glu Ala Pro Ser Gln Val Leu Asn Phe Glu Glu Ile

                20                  25                  30

GAC TAC AAG GAG ATC GAG GTG GAA GAG GTT GTT GGA AGA GGA GCC TTT 323

Asp Tyr Lys Glu Ile Glu Val Glu Glu Val Val Gly Arg Gly Ala Phe

            35                  40                  45

GGA GTT GTT TGC AAA GCT AAG TGG AGA GCA AAA GAT GTT GCT ATT AAA 371

Gly Val Val Cys Lys Ala Lys Trp Arg Ala Lys Asp Val Ala Ile Lys

        50                  55                  60

CAA ATA GAA AGT GAA TCT GAG AGG AAA GCG TTT ATT GTA GAG CTT CGG 419

Gln Ile Glu Ser Glu Ser Glu Arg Lys Ala Phe Ile Val Glu Leu Arg

    65                  70                  75

CAG TTA TCC CGT GTG AAC CAT CCT AAT ATT GTA AAG CTT TAT GGA GCC 467

Gln Leu Ser Arg Val Asn His Pro Asn Ile Val Lys Leu Tyr Gly Ala

80                  85                  90                  95

TGC TTG AAT CCA GTG TGT CTT GTG ATG GAA TAT GCT GAA GGG GGC TCT 515

Cys Leu Asn Pro Val Cys Leu Val Met Glu Tyr Ala Glu Gly Gly Ser

                100                 105                 110

TTA TAT AAT GTG CTG CAT GGT GCT GAA CCA TTG CCA TAT TAT ACT GCT 563
Leu Tyr Asn Val Leu His Gly Ala Glu Pro Leu Pro Tyr Tyr Thr Ala
            115                 120                 125
GCC CAC GCA ATG AGT TGG TGT TTA CAG TGT TCC CAA GGA GTG GCT TAT 611
Ala His Ala Met Ser Trp Cys Leu Gln Cys Ser Gln Gly Val Ala Tyr
        130                 135                 140
CTT CAC AGC ATG CAA CCC AAA GCG CTA ATT CAC AGG GAC CTG AAA CCA 659
Leu His Ser Met Gln Pro Lys Ala Leu Ile His Arg Asp Leu Lys Pro
    145                 150                 155
CCA AAC TTA CTG CTG GTT GCA GGG GGG ACA GTT CTA AAA ATT TGT GAT 707
Pro Asn Leu Leu Leu Val Ala Gly Gly Thr Val Leu Lys Ile Cys Asp
160                 165                 170                 175
TTT GGT ACA GCC TGT GAC ATT CAG ACA CAC ATG ACC AAT AAC AAG GGG 755
Phe Gly Thr Ala Cys Asp Ile Gln Thr His Met Thr Asn Asn Lys Gly
                180                 185                 190
AGT GCT GCT TGG ATG GCA CCT GAA GTT TTT GAA GGT AGT AAT TAC AGT 803
Ser Ala Ala Trp Met Ala Pro Glu Val Phe Glu Gly Ser Asn Tyr Ser
            195                 200                 205
GAA AAA TGT GAC GTC TTC AGC TGG GGT ATT ATT CTT TGG GAA GTG ATA 851
Glu Lys Cys Asp Val Phe Ser Trp Gly Ile Ile Leu Trp Glu Val Ile
        210                 215                 220
ACG CGT CGG AAA CCC TTT GAT GAG ATT GGT GGC CCA GCT TTC CGA ATC 899
Thr Arg Arg Lys Pro Phe Asp Glu Ile Gly Gly Pro Ala Phe Arg Ile
    225                 230                 235
ATG TGG GCT GTT CAT AAT GGT ACT CGA CCA CCA CTG ATA AAA AAT TTA 947
Met Trp Ala Val His Asn Gly Thr Arg Pro Pro Leu Ile Lys Asn Leu
240                 245                 250                 255

CCT AAG CCC ATT GAG AGC CTG ATG ACT CGT TGT TGG TCT AAA GAT CCT 995
Pro Lys Pro Ile Glu Ser Leu Met Thr Arg Cys Trp Ser Lys Asp Pro
                260                 265                 270
TCC CAG CGC CCT TCA ATG GAG GAA ATT GTG AAA ATA ATG ACT CAC TTG 1043
Ser Gln Arg Pro Ser Met Glu Glu Ile Val Lys Ile Met Thr His Leu
            275                 280                 285
ATG CGG TAC TTT CCA GGA GCA GAT GAG CCA TTA CAG TAT CCT TGT CAG 1091
Met Arg Tyr Phe Pro Gly Ala Asp Glu Pro Leu Gln Tyr Pro Cys Gln
        290                 295                 300
TAT TCA GAT GAA GGA CAG AGC AAC TCT GCC ACC AGT ACA GGC TCA TTC 1139
Tyr Ser Asp Glu Gly Gln Ser Asn Ser Ala Thr Ser Thr Gly Ser Phe
    305                 310                 315
ATG GAC ATT GCT TCT ACA AAT ACG AGT AAC AAA AGT GAC ACT AAT ATG 1187
Met Asp Ile Ala Ser Thr Asn Thr Ser Asn Lys Ser Asp Thr Asn Met
320                 325                 330                 335
GAG CAA GTT CCT GCC ACA AAT GAT ACT ATT AAG CGC TTA GAA TCA AAA 1235
Glu Gln Val Pro Ala Thr Asn Asp Thr Ile Lys Arg Leu Glu Ser Lys
                340                 345                 350
TTG TTG AAA AAT CAG GCA AAG CAA CAG AGT GAA TCT GGA CGT TTA AGC 1283
Leu Leu Lys Asn Gln Ala Lys Gln Gln Ser Glu Ser Gly Arg Leu Ser
            355                 360                 365
TTG GGA GCC TCC CAT GGG AGC AGT GTG GAG AGC TTG CCC CCA ACC TCT 1331
Leu Gly Ala Ser His Gly Ser Ser Val Glu Ser Leu Pro Pro Thr Ser
        370                 375                 380
GAG GGC AAG AGG ATG AGT GCT GAC ATG TCT GAA ATA GAA GCT AGG ATC 1379
Glu Gly Lys Arg Met Ser Ala Asp Met Ser Glu Ile Glu Ala Arg Ile
    385                 390                 395

GCC GCA ACC ACA GGC AAC GGA CAG CCA AGA CGT AGA TCC ATC CAA GAC 1427
Ala Ala Thr Thr Gly Asn Gly Gln Pro Arg Arg Arg Ser Ile Gln Asp
400                 405                 410                 415
TTG ACT GTA ACT GGA ACA GAA CCT GGT CAG GTG AGC AGT AGG TCA TCC 1475
Leu Thr Val Thr Gly Thr Glu Pro Gly Gln Val Ser Ser Arg Ser Ser
                420                 425                 430
AGT CCC AGT GTC AGA ATG ATT ACT ACC TCA GGA CCA ACC TCA GAA AAG 1523
Ser Pro Ser Val Arg Met Ile Thr Thr Ser Gly Pro Thr Ser Glu Lys
            435                 440                 445
CCA ACT CGA AGT CAT CCA TGG ACC CCT GAT GAT TCC ACA GAT ACC AAT 1571
Pro Thr Arg Ser His Pro Trp Thr Pro Asp Asp Ser Thr Asp Thr Asn
        450                 455                 460
GGA TCA GAT AAC TCC ATC CCA ATG GCT TAT CTT ACA CTG GAT CAC CAA 1619
Gly Ser Asp Asn Ser Ile Pro Met Ala Tyr Leu Thr Leu Asp His Gln
    465                 470                 475
CTA CAG CCT CTA GCA CCG TGC CCA AAC TCC AAA GAA TCT ATG GCA GTG 1667
Leu Gln Pro Leu Ala Pro Cys Pro Asn Ser Lys Glu Ser Met Ala Val
480                 485                 490                 495
TTT GAA CAG CAT TGT AAA ATG GCA CAA GAA TAT ATG AAA GTT CAA ACA 1715
Phe Glu Gln His Cys Lys Met Ala Gln Glu Tyr Met Lys Val Gln Thr
                500                 505                 510
GAA ATT GCA TTG TTA TTA CAG AGA AAG CAA GAA CTA GTT GCA GAA CTG 1763
Glu Ile Ala Leu Leu Leu Gln Arg Lys Gln Glu Leu Val Ala Glu Leu
            515                 520                 525
GAC CAG GAT GAA AAG GAC CAG CAA AAT ACA TCT CGC CTG GTA CAG GAA 1811
Asp Gln Asp Glu Lys Asp Gln Gln Asn Thr Ser Arg Leu Val Gln Glu
        530                 535                 540

CAT AAA AAG CTT TTA GAT GAA AAC AAA AGC CTT TCT ACT TAC TAC CAG 1859

His Lys Lys Leu Leu Asp Glu Asn Lys Ser Leu Ser Thr Tyr Tyr Gln

    545                 550                 555

CAA TGC AAA AAA CAA CTA GAG GTC ATC AGA AGT CAG CAG CAG AAA CGA 1907

Gln Cys Lys Lys Gln Leu Glu Val Ile Arg Ser Gln Gln Gln Lys Arg

560                 565                 570                 575

CAA GGC ACT TCA TGATTCTCTG GGACCGTTAC ATTTTGAAAT ATGCAAAGAA 1959

Gln Gly Thr Ser

AGACTTTTTT TTTAAGGAAA GGAAAACCTT ATAATGACGA TTCATGAGTG TTAGCTTTTT 2019

GGCGTGTTCT GAATGCCAAC TGCCTATATT TGCTGCATTT TTTTCATTGT TTATTTTCCT 2079

TTTCTCATGG TGGACATACA ATTTTACTGT TTCATTGCAT AACATGGTAG CATCTGTGAC 2139

TTGAATGAGC AGCACTTTGC AACTTCAAAA CAGATGCAGT GAACTGTGGC TGTATATGCA 2199

TGCTCATTGT GTGAAGGCTA GCCTAACAGA ACAGGAGGTA TCAAACTAGC TGCTATGTGC 2259

AAACAGCGTC CATTTTTTCA TATTAGAGGT GGAACCTCAA GAATGACTTT ATTCTTGTAT 2319

CTCATCTCAA AATATTAATA ATTTTTTTCC CAAAAGATGG TATATACCAA GTTAAAGACA 2379

GGGTATTATA AATTTAGAGT GATTGGTGGT ATATTACGGA AATACGGAAC CTTTAGGGAT 2439

AGTTCCGTGT AAGGGCTTTG ATGCCAGCAT CCTTGGATCA GTACTGAACT CAGTTCCATC 2499

CGTAAAATAT GTAAAGGTAA GTGGCAGCTG CTCTATTTAA TGAAAGCAGT TTTACCGGAT 2559

TTTGTTAGAC TAAAATTTGA TTGTGATACA TTGAACAAAA TGGAACTCAT TTTTTTTAAG 2619

GAGTAAAGAT TTTCTTTAGA GCACAATGGA TCTCGAC 2656

## Claims

1. An isolated polypeptide comprising an amino acid sequence from Met at position 1 to Ser at position 579 set forth in SEQ ID NO: 5.

2. An isolated polypeptide consisting of an amino acid sequence from Ser at position 23 to Ser at position 579 set forth in SEQ ID NO: 5.

3. Isolated DNA encoding a polypeptide according to claim 1 or 2.

4. A vector comprising DNA according to claim 3.

5. A host cell transformed with a vector according to claim 4.

6. A method for producing a polypeptide, which comprises culturing a host cell according to claim 5, and then recovering the product from the culture.

7. A polypeptide produced by the method according to claim 6.

8. A fusion protein of a polypeptide according to claim 1 or 2, and another protein.

## Patentansprüche

1. Isoliertes Polypeptid, umfassend eine Aminosäuresequenz von Met an Position 1 bis Ser an Position 579, wie dargestellt in SEQ ID NO: 5.

2. Isoliertes Polypeptid, bestehend aus einer Aminosäuresequenz von Ser an Position 23 bis Ser an Position 579, wie dargestellt in SEQ ID NO: 5.

3. Isolierte DNA codierend ein Polypeptid gemäß Anspruch 1 oder 2.

4. Vektor, umfassend eine DNA gemäß Anspruch 3.

5. Wirtszelle, transformiert mit einem Vektor gemäß Anspruch 4.

6. Verfahren zur Erzeugung eines Polypeptids, das das Kultivieren einer Wirtszelle gemäß Anspruch 5 und dann das Gewinnen des Produkts aus der Kultur umfaßt.

7. Polypeptid, erzeugt durch das Verfahren gemäß Anspruch 6.

8. Fusionsprotein eines Polypeptids gemäß Anspruch 1 oder 2 und eines anderen Proteins.

## Revendications

1. Polypeptide isolé comprenant une séquence en acides aminés de Met en position 1 à Ser en position 579 indiquée dans la séquence dite SEQ ID No:5.

2. Polypeptide isolé consistant en une séquence en acides amines de Ser en position 23 à Ser en position 579, indiquée la séquence dite SEQ ID No:5.

3. ADN isolé codant un polypeptide selon la revendication 1 ou 2.

4. Vecteur comprenant un ADN selon la revendication 3.

5. Cellule hôte transformée avec un vecteur selon la revendication 4.

6. Procédé de production d'un polypeptide, qui comprend la mise en culture d'une cellule hôte selon la revendication 5, puis la récupération du produit dans la culture.

7. Polypeptide produit grâce au procédé selon la revendication 6.

8. Protéine de fusion d'un polypeptide selon la revendication 1 ou 2, et d'une autre protéine.
